# EUROPÄISCHE PATENTSCHRIFT

(11) **EP 0 681 469 B1**
(45) Veröffentlichungstag und Bekanntmachung des Hinweises auf die Patenterteilung: **30.10.1996**
(21) Anmeldenummer: 94905658.4
(22) Anmeldetag: 20.01.1994
(51) Int. Cl.: A61K 7/50, A61K 7/06

(54) **OBERFLÄCHENAKTIVE MISCHUNGEN, DIE EIN TEMPORÄR KATIONISCHES COPOLYMER ENTHALTEN**
MIXTURES OF SURFACTANTS CONTAINING A TEMPORARILY CATIONIC COPOLYMER
MELANGES TENSIOACTIFS CONTENANT UN COPOLYMERE TEMPORAIREMENT CATIONIQUE

(30) Priorität: 28.01.1993 DE 4302315
(43) Veröffentlichungstag der Anmeldung: 15.11.1995
(73) Patentinhaber: Henkel Kommanditgesellschaft auf Aktien, 40191 Düsseldorf (DE)
(72) Erfinder: MÜLLER, Reinhard, D-41812 Erkelenz (DE); SEIDEL, Kurt, D-40589 Düsseldorf (DE); HOLLENBERG, Detlef, D-40699 Erkrath (DE); EHLERT, Manuela, D-51379 Leverkusen (DE)
(86) Internationale Anmeldenummer: EP9400133
(87) Internationale Veröffentlichungsnummer: WO9416679

(56) Entgegenhaltungen:
- DE-A- 4 207 046
- DE-U- 9 110 351
- GB-A- 2 117 784
- SOAP, COSMET. CHEM. SPEC. Bd. 56, Nr. 2 , 1980 , NJ, US Seite 34 E. J. MURPHY 'CATIONIC POLYMER CONDITIONER EVALUATION'

## Beschreibung

### Gebiet der Erfindung

Die Erfindung betrifft oberflächenaktive Mischungen, insbesondere Mittel zum Waschen oder Spülen von Haaren, die aufgrund ihrer Zusammensetzung besonders hautschonend sind.

### Stand der Technik

Zur Erzielung einer reinigenden Wirkung enthalten wäßrige Reinigungsmittel üblicherweise oberflächenaktive Verbindungen, wodurch in der Regel die Hautbelastung des Mittels erhöht wird. Dies gilt besonders für die wichtige Klasse der Aniontenside.

Diese erhöhte Hautbelastung sollte im Bereich der Körperreinigungsmittel vermieden werden. Insbesondere bei Produkten, die für eine häufige Anwendung konzipiert sind, zur Reinigung des Intimbereiches dienen oder mit Schleimhäuten in Berührung kommen, ist eine gute Hautverträglichkeit wichtig. Es besteht daher ein ständiger Bedarf an milden wäßrigen Reinigungsmitteln mit gutem Schaumvermögen.

Von besonderem Interesse auf dem Gebiet der wäßrigen Reinigungsmittel sind Mittel zum Waschen oder Spülen von Haaren, zum Beispiel Shampoos und abspülbare Haarnachbehandlungsmittel. Dabei besteht insbesondere ein ständiger Bedarf nach solchen Mitteln, die eine verbesserte Wirkung hinsichtlich Fülle und Frisierbarkeit der Haare aufweisen.

Das Haupthaar weist nach dem Waschen oft einen kosmetisch unbefriedigenden Zustand auf. Es fühlt sich stumpf an, ist im nassen Zustand nur schwer zu kämmen und neigt im trockenen Zustand zur statischen Aufladung wodurch das Kämmen erschwert und der Sitz des gekämmten Haares gestört wird.

Bekannt ist die Verwendung zwitterionischer Polymerer, die anionische Gruppen, in der Regel Carboxylgruppen, und quartäre Ammoniumgruppen im Molekül enthalten, in Haarbehandlungsmitteln. Beispielsweise beschreibt die DE-OS-21 50 557 die Verwendung von Polymerisaten zwitterionischer Monomerer in Haarfestlegemittel. Zwitterionische Polymere zeigen jedoch insbesondere in Formulierungen mit anionischen Tensiden den Nachteil, daß die haaravivierenden und haarfestigenden Eigenschaften im Laufe längerer Lagerzeit allmählich verloren gehen.

Die deutsche Patentschrift DE 33 36 760 C2 (L'Oreal) beschreibt milde Reinigungs-Zusammensetzungen enthaltend Glucosid-Alkylether mit Alkylresten der Kettenlänge C₈₋₁₀ und nichtionische Polymere wie Polyvinylpyrrolidon bzw. desssen Copolymeren mit nichtionischen Co-Monomeren.

Es ist weiterhin bekannt, nach dem Waschen oder Shampoonieren des Haares konditionierende Präparate, meist auf Basis kationischer Tenside, einwirken zu lassen oder den Haarwaschmitteln konditionierende Stoffe zuzusetzen, um gleichzeitig mit der Haarwäsche einen gewissen konditionierenden Effekt zu erzielen. Solche Substanzen sind zum Beispiel kationische Polymere wie kationische Cellulosederivate.

So beschreibt die europäischen Patentanmeldung EP 337 354 (Kao) Mittel, die eine Kombination von Alkylglykosiden und mindestens einem kationischen Polymer enthalten. Mit dieser Kombination sollen gemäß Aufgabenstellung der EP 337 354 Mittel erhalten werden, die hautschonend sind und ein gewisses Schaumvermögen aufweisen.

Bei den gemäß der Lehre der EP 337 354 einzusetzenden kationischen Polymeren handelt es sich im wesentlichen um Substanzen, die einen permanenten kationischen Charakter haben, d.h. deren molekulare Struktur so zu beschreiben ist, daß sie einerseits einen polymeren Teil mit permanenten kationischen Zentren aufweist, andererseits entsprechende Gegenionen anionischen Charakters. Unter "permanent" wird dabei verstanden, daß die positive Ladungen an den Heteoratomen wie Stickstoff zeitlich unveränderlich fixiert ist. Als typisches Beispiel seien die weithin bekannten quaternären Ammoniumsalze angeführt, bei denen der Stickstoff mit vier organischen Resten verknüpft ist. Die positive Ladung des Stickstoffs hat daher permanenten Charakter und bleibt solange erhalten, als die Verbindung nicht chemisch modifiziert, d.h. der Substitutionsgrad des Stickstoffs durch chemische Reaktion verändert wird.

### Beschreibung der Erfindung

Das Anforderungsprofil, das an oberflächenaktive Mischungen der erfindungsgemäß betroffenen Art gestellt wird, beschränkt sich jedoch nicht auf die geforderten hautschonenden Eigenschaften sowie ein gewisses Schaumvermögen, es sind vielmehr eine Vielzahl weiterer Forderungen zu erfüllen.

Mit den aus dem Stand der Technik - insbesondere der EP 337 354 - bekannten Mitteln wird zwar eine befriedigende Verbesserung der Naßkämmbarkeit und auch eine Verringerung der statischen Aufladung erzielt. Diese Effekte gehen jedoch praktisch immer mit einer zu starken Glättung des trockenen Haares einher. Dadurch entsteht der Nachteil, daß das Haar wenig Fülle und die Frisur keinen Halt aufweist. Die Glätte des Haares ist um so ausgeprägter, je niedriger der Kämmwiderstand des trockenen Haares ist.

Die Anmelderin hat darüber hinaus beobachtet, daß wäßrige Zusammensetzungen auf Basis permanenter kationischer Polymere in der Regel trübe sind, eine Tatsache, die unter anwendungstechnischen Gesichtspunkten unerwünscht ist, da hier klare Produkte favorisiert werden.

Es bestand daher die Aufgabe, oberflächenaktive Mischungen und insbesondere Mittel zum Waschen und Spülen von Haaren, aufzufinden, welche bei gleichzeitig gutem Schaumvermögen und geringer Hautbelastung die Frisierbarkeit und den Frisurenhalt des Haares verbessern, ohne eine Klebrigkeit des Haares zu verursachen. Darüber hinaus sollten diese Mischungen keine Neigung zur Austrübung aufweisen, sondern in klarer Form vorliegen.

Es wurde nun überraschend gefunden, daß Fülle, Frisierbarkeit und Frisurenhalt der Haare im Vergleich zum bekannten Stand der Technik deutlich verbessert werden, wenn die oberflächenaktive Mischung 1 bis 50 Gew.-% eines Aniontensids, 0,5 bis 15 Gew.-% eines Alkylglykosids und 0,1 bis 7 Gew.-% eines speziellen temporären kationischen Copolymers enthält.

Gegenstand der Erfindung sind dementsprechend oberflächenaktive Mischungen enthaltend
(A) 1 bis 50 Gew.-% eines oder mehrerer Aniontenside, die 1 oder 2 lipophile Reste mit 1 bis 22 C-Atomen und einen polaren Rest ausgewählt aus der Gruppe der Carboxylat-, Sulfat- oder Sulfonatreste und gegebenenfalls einen Polyoxyalkylenrest mit einem mittleren Alkoxylierungsgrad von 1 bis 15 enthalten,
(B) 0,5 bis 15 Gew.-% eines oder mehrerer Alkylglycoside der allgemeinen Formel R(G)ₓ worin R einen linearen, gesättigten Alkylrest mit 8 bis 22 C-Atomen und (G)ₓ ein Glycosid- oder Oligoglycosidrest mit einem Oligomerisationsgrad x von 1 bis 10 ist,
(C) 0,1 bis 7 Gew.-% eines stickstoffhaltigen Copolymeren mit einem Molekulargewicht von etwa 15.000 bis 1.500.000, das
   (i) 99,5 bis 45 mol.-% von Vinylpyrrolidon hergeleitete Einheiten,
   (ii) 0,5 bis 50 mol.-% von einem Monomeren der Formel (I)

      CH₂=CHR¹-COOR²-NR³R⁴ (I)

      worin
      - R¹ Wasserstoff oder Methyl,
      - R² eine Alkylengruppe mit 1 bis 20 C-Atomen und
      - R³ und R⁴ unabhängig voneinander eine Alkylgruppe mit 1 bis 4 C-Atomen bedeuten,
      hergeleitete Einheiten und
   (iii) 0 bis 50 mol-% von Einheiten, die von mindestens einem - von (i) und (ii) verschiedenen - ethylenisch ungesättigten, mischpolymerisierbaren Monomeren hergeleitet sind, umfaßt, und
(D) 28 bis 98,4 Gew.-% Wasser,
   mit der Maßgabe, daß
   a) der pH-Wert der Mischung 3 bis 7 beträgt, so daß das Polymere (C) in temporär kationischer Form vorliegt,
   b) die Summe der Komponenten (B) und (C) und nicht größer als der Gehalt an Komponente (A) ist und
   c) die Mischung frei ist von stickstoffhaltigen Polymeren und/oder Copolymeren mit permanent kationischem Charakter.

Unter Polyoxyalkylenrest wird dabei eine Gruppe verstanden, die aus Oxyethylen-Einheiten -[CH₂-CH₂-O]- oder aus Oxypropylen-Einheiten -[CH(CH₃)-CH₂-O]- aufgebaut ist. Wie in der Fachwelt allgemein üblich wird dabei unter dem mittleren Alkoxylierungsgrad die mittlere Anzahl der Oxyalkylen-Einheiten pro Molekül des Aniontensids verstanden.

Die **Aniontenside (A)** werden erfindungsgemäß bevorzugt ausgewählt aus der Gruppe der Alkyl- und Dialkylethersulfate, Ethercarbonsäuren, Sulfobernsteinsäurehalbester, Fettalkoholethercitrate, Fettalkoholethertartrate, Acylsarkoside, Acyltauride, Acylisethionate und der Sulfonate von ungesättigten Fettsäuren.

Die Gegenionen der Carboxylat-, Sulfat oder Sulfonatreste werden bevorzugt aus der Gruppe der Alkali- und Erdalkalimetalle, Aluminium, Ammonium sowie Alkyl- oder Alkylolammoniumgruppen mit 1 bis 4 C-Atomen in jeder Alkyl- oder Alkylolgruppe gewählt. Ganz besonders geeignet ist die Gruppe der Alkalimetalle.

Die chemischen Strukturen sowie die grundlegenden Tensideigenschaften der meisten dieser Aniontenside gehören mittlerweile zum Lehrbuchwissen und bedürfen daher keiner weiteren Erläuterung. Weiter geeignete Aniontenside seien im folgenden genannt: Unter Dialkylethersulfaten sind Verbindungen zu verstehen wie sie in der Europäischen Patentameldung EP 299 370 beschrieben sind. Aus dieser Schrift können Einzelheiten des Herstellungsverfahrens und der Eigenschaften dieser Verbindungen entnommen werden. Unter Fettalkoholethertartraten sind Monoestersalze der Weinsäure, unter Fettalkoholethercitraten Mono-und/oder Diestersalze der Zitronensäure mit Anlagerungsprodukten von Ethylenoxid und/oder Propylenoxid an Fettalkohole zu verstehen. Unter Sulfonaten von ungesättigten Fettsäuren sind Sulfonierungsprodukte von Fettsäuren mit 12 bis 22 C-Atomen und 1 bis 6 Doppelbindungen zu verstehen. Derartige Produkte sind literaturbekannt und beispielsweise durch Umsetzung dieser Fettsäuren mit gasförmigem Schwefeltrioxid zugänglich. Am Beispiel der Ölsäure können Einzelheiten des Herstellungsverfahrens der deutschen Patentanmeldung DE 39 26 344 entnommen werden.

Bei Aniontensiden, die einen Polyoxyalkylenrest enthalten, gilt bezüglich des Alkoxylierungsgrades ganz allgemein, daß bei Alkoxylierungsreaktionen wie beispielsweise der Anlagerung von x mol Ethylenoxid an 1 mol Fettalkohol nach den bekannten Ethoxylierungsverfahren kein einheitliches Addukt, sondern ein Gemisch aus Restmengen freien Fettalkohols und einer Reihe homologer (oligomerer) Anlagerungsprodukte von 1, 2, 3, ... x, x+1, x+2 ... usw. Molekülen Ethylenoxid je Molekül Fettalkohol erhalten wird. Der mittlere Ethoxylierungsgrad (x) wird dabei definiert durch die Ausgangsmengen an Fettalkohol und Ethylenoxid. Die Verteilungskurve des Homologengemisches weist in der Regel ein Maximum im Bereich zwischen x-3 und x+3 auf. Nähere Informationen hierzu können beispielsweise der Zeitschrift Soap/Cosmetics/Chemical Specialities, Heft Januar 1988, S. 34, entnommen werden. Neben den üblichen aus dem Stand der Technik bekannten Alkoxylierungskatalysatoren wie Natriummethanolat lassen sich dabei aber auch solche Katalysatoren verwenden, die zu Produkten mit sogenannter eingeengter Homologenverteilung führen, vergl. z.B. Seifen-Öle-Fette-Wachse 1990 (116) 60.

In einer bevorzugten Ausführungsform der Erfindung beträgt der Anteil der anionischen Tenside 4 bis 30 Gew.-%.

**Alkylglycoside (B)** der allgemeinen Formel R-(G)ₓ sind seit langem bekannte oberflächenaktive Stoffe, die aus Zuckern und aliphatischen, primären Alkoholen mit 8 - 22 C-Atomen unter Acetalisierung herstellbar sind. Als Zuckerkomponenten (Glycosen) kommen bevorzugt Glucose, daneben aber auch Fructose, Mannose, Galactose, Telose, Gulose, Allose, Altrose, Idose, Arabinose, Xylose, Lyxose, Libose und Gemische davon in Frage.

Bevorzugt wegen der leichten Zugänglichkeit und der guten Anwendungseigenschaften sind die Acetalisierungsprodukte der Glucose mit Fettalkoholen R-OH, die z. B. aus natürlichen Fetten und Ölen nach bekannten Verfahren erhältlich sind, insbesondere mit linearen, primären, gesättigten und ungesättigten Fettalkoholen mit 8 bis 22 C-Atomen.

Alkylglycoside, ihre Herstellung und Verwendung als oberflächenaktive Stoffe sind beispielsweise aus US-A-3.839.318, US-A-3.707.535, US-A-3.547.828, DE-A-1943689, DE-A-2036472, DE-A-3001064 sowie EP-A-77167 bekannt`. Bezüglich des Glycosidrestes - (G)ₓ gilt, daß sowohl Monoglycoside (x = 1), bei denen ein Zuckerrest glycosidisch mit dem Fettalkohol verbunden ist, als auch oligomere Glycoside mit einem Oligomerisationsgrad x = 2 bis 10 geeignet sind. In der Regel liegen Gemische von Mono- und Oligoglycosiden vor.

Bevorzugt geeignet sind solche Alkylglycoside (B), in welchen R eine Alkylgruppe mit 8 bis 22 C-Atomen und (G)ₓ ein Glycosid- oder Oligoglycosidrest mit einem Oligomerisationsgrad x = 1 bis 10 ist. Ganz besonders bevorzugt ist R eine Alkylgruppe mit 10 bis 16 C-Atomen, und (G)ₓ, der Rest eines Gemisches von Glucosid und Oligoglucosiden mit einem mittleren Oligomerisationsgrad von 1 bis 1,5.

In eine bevorzugten Ausführungsform der Erfindung beträgt der Anteil des Alkylglykosids 1 bis 8 Gew.-%.

Wie bereits ausgeführt eignen sich gemäß der Lehre der vorliegenden Erfindung nur solche **stickstoffhaltigen Copolymere (C)**, die bei einem pH-Wert des Mittels im Bereich von 3 bis 7 in temporär kationischer Form vorliegen. Ausdrücklich sei an dieser Stelle nochmals darauf hingewiesen, daß stickstoffhaltige Copolymere, deren N-Atome zumindest teilweise in permanent kationischer Form vorliegen, nicht zu den erfindungsgemäß einzusetzenden Copolymeren (C) zu rechnen sind.

In Anlehnung an den oben erläuterten Begriff eines "permanenten" kationischen Polymeren sei in diesem Zusammenhang klargestellt, daß eine "temporär" kationische Form eines stickstoffhaltigen Copolymeren dann vorliegt, wenn sich das Ausmaß der positiven Ladung der Stickstoffatome in einfacher Weise, etwa durch Einstellung des pH-Wertes der wäßrigen Mischung modifizieren läßt. Strukturell wird dies dadurch ermöglicht, daß die Stickstoffatome der entsprechenden Polymeren nur mit drei organischen Resten verknüpft sind. Durch Einstellung eines sauren pH-Wertes wird dann der Stickstoff positiv geladen; diese Ladung läßt sich jedoch durch Änderung des pH-Wertes in Richtung eines alkalischen Milieus wieder entfernen, ohne daß die Struktur des Polymers dabei verändert wird.

Als besonders geeignet haben sich diejenigen Copolymeren (C) von Vinylpyrrrolidon und Dimethylaminoethylmethacrylat erwiesen, die durch die Struktur (I-a) 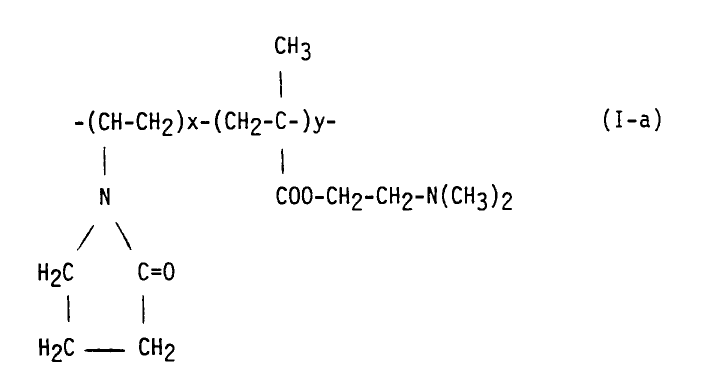 gekennzeichnet sind und worin x und y innerhalb der oben näher bezeichneten Grenzen so gewählt sind, daß die Verbindungen ein Molgewicht im Bereich von 15.000 bis 1.500.000 aufweisen. Derartige Produkte sind teilweise kommerziell erhältlich. Als Beispiel sei das "Copolymer 937" genannt.

In einer bevorzugten Ausführungsform der Erfindung beträgt der Anteil des temporären kationischen Polymers (C) 0,5 bis 5 Gew.-%.

Weiterhin können die erfindungsgemäßen Mittel neben den Aniontensiden (A) zusätzlich 0,5-20 Gew.-%, insbesondere 1-10 Gew.-%, an ampholytischen und/oder zwitterionischen Tensiden enthalten.

Unter **ampholytischen Tensiden** werden solche oberflächenaktiven Verbindungen verstanden, die außer einer C₈-C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarkosine, 2-Alkylaminopropionsäuren und Alkylaminoessigsäuren mit jeweils etwa 8 bis 18 C-Atomen in der Alkylgruppe.

Als **zwitterionische Tenside** werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinat, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Die hautschonenden Eigenschaften der erfindungsgemäßen Mittel kommen besonders dann zur Geltung, wenn diese so formuliert werden, daß sie einen pH-Wert in der Nähe des Neutralpunktes der Haut aufweisen. Mittel mit pH-Werten im Bereich von 5,0 - 6,5 sind dabei bevorzugt.

Die erfindungsgemäßen Mittel können darüber hinaus **anorganische Elektrolytsalze (E)** enthalten. Dazu eignen sich alle wasserlöslichen Alkali-, Ammonium- und Erdalkalisalze, z. B. die Fluoride, Chloride, Bromide, Sulfate, Phosphate und Nitrate und Hydrogencarbonate, soweit sie in einer Menge von wenigstens 1 Gew.-% bei 20°C in Wasser löslich sind. Bevorzugt werden die Chloride oder Sulfate eines Alkalimetalls, des Ammoniums oder des Magnesiums verwendet; besonders bevorzugt sind Natriumchlorid und Magnesiumchlorid. Bevorzugt beträgt die Menge des Elektrolytsalzes 0,1 bis 10 Gew.-%.

Die erfindungsgemäßen Mittel können in einer Vielzahl von reinigenden und/oder pflegenden Konsumentenprodukten Verwendung finden. Dazu zählen beispielsweise kosmetische Mittel wie Haarshampoos, Schaumbädern, Duschbädern, flüssigen Seifen, abspülbare Haarnachbehandlungsmittel wie Haarspülungen, Haarkuren und dergleichen. Insbesondere eignen sie sich für milde Haarshampoos mit verbesserter Fülle und Frisierbarkeit der Haare. Die erfindungsgemäßen Mittel können aber auch in manuellen Geschirrspülmitteln Verwendung finden.

Diese Produkte enthalten neben Tensiden oder Tensidkombinationen üblicherweise Bestandteile wie Emulgatoren, Ölkomponenten, Lösungsvermittler. Verdickungsmittel, Überfettungsmittel, biogene Wirkstoffe, Filmbildner, Duftstoffe, Farbstoffe, Perlglanzmittel, Schaumstabilisatoren, Konservierungsmittel und pH-Regulatoren. Die erfindungsgemäßen Mittel können daher zusätzliche Komponenten und Hilfsstoffe enthalten, wie sie aus dem Stand der Technik bekannt sind. Die wichtigsten sind:
**Nichtionische Tenside/Emulgatoren**, z.B. Ethylenoxidaddukte an Alkohole, Carbonsäuren, Partialglyceride und Sorbitanester sowie z.B. Fettsäureester und Sorbitanfettsäureester (vergl. z.B. W. Umbach [Hrsg.], "Kosmetik - Entwicklung, Herstellung und Anwendung kosmetischer Mittel", S.86-87, Stuttgart 1988).
**Ölkomponenten**, z.B. Substanzen wie Paraffinöl, Pflanzenöle, Fettsäureester, Squalan und 2-Octyldodecanol; als Fette und Wachse beispielsweise Walrat, Bienenwachs, Montanwachs, Paraffin und Cetyl-stearylalkohol.
**Lösungsvermittler**, z.B. niedrige ein- oder mehrwertige Alkohole wie Ethanol, Isopropanol, Ethylenglykol, Propylenglykol, Glycerin, 1,3-Butylenglykol und Diethylenglykol.
**Verdickungsmittel**, z.B. Polysaccharide, insbesondere Xanthan-Gum, Guar-Gum, Agar-Agar, Alginate und Tylosen, sowie Carboxymethylcellulose und Hydroxyethylcellulose, ferner höhermolekulare Polyethylenglykolmono- und -diester von Fettsäuren, Polyacrylate, Polyvinylalkohol und Polyvinylpyrrolidon.
**Überfettungsmittel**, z.B. polyethoxylierte Lanolinderivate, Lecithinderivate und Fettsäurealkanolamide, wobei die letzteren gleichzeitig auch als Schaumstabilisatoren dienen.
**Biogenen Wirkstoffe** wie Pflanzenextrakte, Eiweißabbauprodukte und Vitaminkomplexe.
**Feuchthaltmittel**, z.B. Glycerin, Polyglycerine, Sorbit, 1,2-Propandiol, 1,2,3-Butan-triol, Polyethylenglykole, Glucose, Mannit, Xylit, Pyrollidon-Carbonsäure-Salze (PCA), Aminosäuren, Milchsäure.
**Antimikrobiell wirksame Stoffe** als Konservierungsmittel, z.B. Benzoesäure, Salicylsäure, Sorbinsäure, sowie deren Ester und Salze sowie die in der Anlage zur Kosmetikverordnung aufgeführten Substanzen.
**Perlglanzmittel** wie Glykoldistearinsäureester, Ethylenglykoldistearat oder Fettsäuremonoglykolester.
**Duftstoffe**, z.B. natürliche Riechstoffe, die durch Destillation, Extraktion oder Pressung aus Pflanzen gewonnen werden sowie synthetisch hergestellte Riechstoffe (vergl. z.B. H.Aebi, E.Baumgartner, H.P.Fiedler, G.Ohloff, "Kosmetika, Riechstoffe und Lebensmittelzusatzstoffe", Stuttgart 1978).
**Antioxidantien**, z.B. Tocopherole, Lecithin, Guajakol, Butylkresol, 4-Methyl-2,6-di-tert.-butyl-phenol (BHT), 4-Methoxy-2(3)tert.-butylphenol (BHA)
**Farbstoffe**, wie sie z.B. von der Farbstoff-Kommission der Deutschen Forschungsgemeinschaft für Kosmetika zusammengestellt sind ("Färbemittel für Kosmetika" Mitteilung 3, Wiesbaden 1968). Die Farbstoffe werden üblicherweise in Konzentrationen von 0,001 bis 0,1 Gew.-%, bezogen auf die gesamte Mischung, eingesetzt.
**pH-Regulatoren:** Weitere Komponenten der erfindungsgemäßen Mittel sind gewünschtenfalls Substanzen, die der Einstellung des pH-Wertes der Mittel dienen.

Die Gesamtmenge der Hilfsstoffe beträgt 0 - 20 Gew.-%, vorzugsweise 0 - 10 Gew.-%.

Zur Herstellung der erfindungsgemäßen Mischungen wird das Alkylglycosid (B) bei 60 bis 80 °C zu einer wäßrigen Phase gegeben, die die Aniontenside (A) und das synthetische nichtionische Polymer (C) enthält. Diese Mischungen werden gerührt und dann auf Normaltemperatur (20 bis 40 °C) abgekühlt. Wegen der Wasserlöslichkeit der Alkylglycoside, insbesondere solcher mit Alkylresten von 12 bis 16 C-Atomen, können die erfindungsgemäßen Mittel vorteilhaft auch auf kaltem Wege hergestellt werden. Dabei werden die Komponenten in einfacher Weise bei Normaltemperatur zusammengerührt.

Die nachfolgenden Beispiele dienen der Erläuterung der Erfindung.

### Beispiele

### 1. Allgemeines

### 1.1. Abkürzungen

In der Kopfzeile der Tabellen 1 und 2 ist jeweils das erfindungsgemäße Beispiel mit **B1**, der Vergleich mit **V1** kenntlich gemacht.

### 1.2. Verwendete Substanzen

### 1.2.1. Tenside

**Dehyton K**: Wäßrige Lösung eines Fettsäureamid-Derivats mit Betainstruktur der Formel R-CONH-(CH₂)₃-N⁺(CH₃)₂-CH₂-COO⁻ ; CTFA-Bezeichnung: Cocamidopropyl Betaine; Aktivsubstanzgehalt: 30 Gew.-%; NaCl-Gehalt: 5 Gew.-% (Fa. Henkel/Düsseldorf)
**AKYP**: Wäßrige Lösung eines Ethercarbonsäuresalzes der Formel C_{12/14}-(O-CH₂-CH₂)₁₀-OCH₂-COONa, Aktivsubstanzgehalt: 22 Gew. % ("Akypo^{(R)}-Soft 100 NV"; Fa. Chemy-Y)
**APG600**: C_{12/14}-Fettalkoholglucosid mit einem Oligomerisationsgrad von 1,45 (Fa. Henkel/Düsseldorf).
**Texapon K14S:** Wäßrige Lösung von Natriumlaurylmyristylethersulfat; Aktivsubstanzgehalt: 28 Gew.-% ("Texapon^{(R)} K14S"; Fa. Henkel/Düsseldorf)

### 1.2.2. Polymere

**COPOLY-1:** Copolymer von Vinylpyrrolidon und Dimethylaminoethylmethacrylat; Molgewicht ca. 1.000.000 ("Copolymer 937"; Fa. GAF)
**COPOLY-2:** Copolymer von Hydroxyethylcellulose und Diallyldimethylammoniumchlorid, CTFA Bezeichnung Polyquaternium-4; 95 % Aktivsubstanzgehalt ("Celquat L 200"; Fa. Delft National)

### 1.2.3. Sonstige Stoffe

**Nutrilan I:** Eiweiß-Hydrolysat; CTFA-Bezeichnung: hydrolyzed-animal-collagen ("Nutrilan^{(R)} I"; Fa. Grünau/Illertissen)

### 2. Bestimmung der Naßkämmbarkeit (Untersuchungsmethode)

Den Untersuchungen zur Kämmbarkeit wurde die Methode gemäß J. Soc. Cosm. Chem. 1973 [24] 782 zugrundegelegt.

Es wurde jeweils die Kämmbarkeit an braunem Haar (Alkinco #6634, Strähnenlänge 12 cm, Strähnenmasse 1 g) untersucht. Es handelte sich um leicht vorgeschädigte (kaltgewellte bzw. blondierte) Haare, wie sie etwa beim durchschnittlichen Anwender zu erwarten sind. Nach der Nullmessung wurden die Strähnen mit 100 ml der zu prüfenden Rezeptur getränkt. Nach 5 Minuten Einwirkzeit wurden die Strähnen 1 Minute unter fließendem Wasser (1 l/min, 38 °C) ausgespült. Zur Bestimmung der Naßkämmbarkeit wurden die Strähnen dann erneut vermessen.

### 3. Versuchsergebnisse

Für die nachstehend in Tabelle 1 beschriebenen Zusammensetzungen V1 und B1 wurden die Naßkämmbarkeit sowie das Aussehen der Zusammensetzung untersucht. Die Angaben in der Tabelle 1 beziehen sich dabei auf Gew.-% Aktivsubstanz.

Die Untersuchungen wurden von einem Versuchspanel von 6 Personen, die über praktische Erfahrungen bei der Beurteilung von Haarsträhnen verfügten, durchgeführt. Die Ergebnisse der Naßkämmbarkeitsuntersuchungen wurden auf einer Skala von 1 (sehr gut) bis 5 (sehr schlecht) wiedergegeben.

Es wurde gefunden (vergl. Tabelle 2), daß die erfindungsgemäße Zusammensetzung B1 dem Vergleichsbeispiel V1
a) hinsichtlich der Naßkämmbarkeit überlegen
   und
b) von klarem (im Gegensatz zu trübem) Aussehen ist.

**Tabelle 1**

| **Zusammensetzungen V1 und B1** | | |
|---|---|---|
| Substanz | V1 | B1 |
| Texapon K14S | 42,0 | 42,0 |
| Dehyton K | 10,0 | 10,0 |
| Nutrilan I | 0,5 | 0,5 |
| APG 600 | 2,0 | 2,0 |
| AKYP | 5,0 | 5,0 |
| Copoly-1 | - | 2,0 |
| Copoly-2 | 2,0 | - |
| Na-Salicylat | 0,57 | 0,57 |
| NaCl | 1,0 | 1,0 |
| Wasser | ad 100 | ad 100 |

**Tabelle 2**

| **Versuchsergebnisse V1 und B1** | | |
|---|---|---|
| | V1 | B1 |
| Naßkämmbark. | 4 | 3 |
| Aussehen | trüb | klar |

## Patentansprüche

1. Oberflächenaktive Mischungen enthaltend
(A) 1 bis 50 Gew.-% eines oder mehrerer Aniontenside, die 1 oder 2 lipophile Reste mit 1 bis 22 C-Atomen und einen polaren Rest ausgewählt aus der Gruppe der Carboxylat-, Sulfat- oder Sulfonatreste und gegebenenfalls einen Polyoxyalkylenrest mit einem mittleren Alkoxylierungsgrad von 1 bis 15 enthalten,
(B) 0,5 bis 15 Gew.-% eines oder mehrerer Alkylglycoside der allgemeinen Formel R(G)ₓ worin R einen linearen, gesättigten Alkylrest mit 8 bis 22 C-Atomen und (G)ₓ ein Glycosid- oder Oligoglycosidrest mit einem Oligomerisationsgrad x von 1 bis 10 ist,
(C) 0,1 bis 7 Gew.-% eines stickstoffhaltigen Copolymeren mit einem Molekulargewicht von etwa 15.000 bis 1.500.000, das
(i) 99,5 bis 45 mol.-% von Vinylpyrrolidon hergeleitete Einheiten,
(ii) 0,5 bis 50 mol.-% von einem Monomeren der Formel (I)
CH₂=CHR¹-COOR²-NR³R⁴ (I)
worin
- R¹ Wasserstoff oder Methyl,
- R² eine Alkylengruppe mit 1 bis 20 C-Atomen und
- R³ und R⁴ unabhängig voneinander eine Alkylgruppe mit 1 bis 4 C-Atomen bedeuten,
hergeleitete Einheiten und
(iii) 0 bis 50 mol-% von Einheiten, die von mindestens einem - von (i) und (ii) verschiedenen - ethylenisch ungesättigten, mischpolymerisierbaren Monomeren hergeleitet sind,
umfaßt, und
(D) 28 bis 98,4 Gew.-% Wasser,
mit der Maßgabe, daß
a) der pH-Wert der Mischung 3 bis 7 beträgt, so daß das Polymere (C) in temporär kationischer Form vorliegt,
b) die Summe der Komponenten (B) und (C) und nicht größer als der Gehalt an Komponente (A) ist und
c) die Mischung frei ist von stickstoffhaltigen Polymeren und/oder Copolymeren mit permanent kationischem Charakter.

2. Oberflächenaktive Mischungen nach Anspruch 1, wobei Copolymer (C) ein Copolymer von Vinylpyrrrolidon und Dimethylaminoethylmethacrylat ist.

3. Oberflächenaktive Mischungen nach Anspruch 1 oder 2, worin der Anteil des Aniontensids (A) 4 bis 30 Gew.-% beträgt.

4. Oberflächenaktive Mischungen nach einem der Ansprüche 1 bis 3, worin der Anteil des Alkylglykosids (B) 1 bis 8 Gew.-% beträgt.

5. Oberflächenaktive Mischungen nach einem der Ansprüche 1 bis 4, worin der Anteil des synthetischen nichtionischen Polymers (C) 0,5 bis 5 Gew.-% beträgt.

6. Oberflächenaktive Mischungen nach einem der Ansprüche 1 bis 5, die zusätzlich 0,1 bis 10 Gew.-% eines anorganischen Elektrolytsalzes (E) enthalten.

7. Verwendung der oberflächenaktiven Mischungen nach einem der Ansprüche 1 bis 6 in abspülbaren kosmetischen Formulierungen.

8. Verwendung der oberflächenaktiven Mischungen nach einem der Ansprüche 1 bis 6 in reinigenden kosmetischen Produkten wie Haarshampoos, Schaumbädern, Duschbädern, flüssigen Seifen und dergleichen.

9. Verwendung der oberflächenaktiven Mischungen nach einem der Ansprüche 1 bis 6 in pflegenden kosmetischen Produkten wie Haarspülungen, Haarkuren und dergleichen.

10. Verwendung der oberflächenaktiven Mischungen nach einem der Ansprüche 1 bis 6 in Spül- und Reinigungsmitteln.

## Claims

1. Surface-active mixtures containing
(A) 1 to 50% by weight of one or more anionic surfactants containing one or two lipophilic groups containing 1 to 22 carbon atoms and a polar group selected from carboxylate, sulfate or sulfonate groups and optionally a polyoxyalkylene group with an average degree of alkoxylation of 1 to 15,
(B) 0.5 to 15% by weight of one or more alkyl glycosides corresponding to the general formula R(G)ₓ, where R is a linear saturated alkyl radical containing 8 to 22 carbon atoms and (G)ₓ is a glycoside or oligoglycoside unit with a degree of oligomerization x of 1 to 10,
(C) 0.1 to 7% by weight of a nitrogen-containing copolymer with a molecular weight of around 15,000 to 1,500,000 which contains
(i) 99.5 to 45 mole-% of units derived from vinyl pyrrolidone,
(ii) 0.5 to 50 mole-% of units derived from a monomer corresponding to formula (I):
CH₂=CHR¹-COOR²-NR³R⁴ (I)
in which
- R¹ is hydrogen or methyl,
- R² is a C₁₋₂₀ alkylene group and
- R³ and R⁴ independently of one another represent a C₁₋₄ alkyl group,
and
(iii) 0 to 50 mole-% of units derived from at least one ethylenically unsaturated copolymerizable monomer different from (i) and (ii), and
(D) 28 to 98.4% by weight of water,
with the proviso that
a) the pH value of the mixture is in the range from 3 to 7 so that the polymer (C) is present in temporarily cationic form,
b) the sum of components (B) and (C) is no greater than the content of component (A) and
c) the mixture is free from nitrogen-containing polymers and/or permanently cationic copolymers.

2. Surface-active mixtures as claimed in claim 1, in which copolymer (C) is a copolymer of vinyl pyrrolidone and dimethylaminoethyl methacrylate.

3. Surface-active mixtures as claimed in claim 1 or 2, in which the percentage content of the anionic surfactant (A) is 4 to 30% by weight.

4. Surface-active mixtures as claimed in any of claims 1 to 3, in which the percentage content of the alkyl glycoside (B) is 1 to 8% by weight.

5. Surface-active mixtures as claimed in any of claims 1 to 4, in which the percentage content of the synthetic nonionic polymer (C) is 0.5 to 5% by weight.

6. Surface-active mixtures as claimed in any of claims 1 to 5 additionally containing 0.1 to 10% by weight of an inorganic electrolyte salt (E).

7. The use of the surface-active mixtures claimed in any of claims 1 to 6 in rinsable cosmetic formulations.

8. The use of the surface-active mixtures claimed in any of claims 1 to 6 in cleaning cosmetic products, such as hair shampoos, foam baths, shower baths, liquid soaps and the like.

9. The use of the surface-active mixtures claimed in any of claims 1 to 6 in cosmetic personal-care products, such as hair rinses, hair tonics and the like.

10. The use of the surface-active mixtures claimed in any of claims 1 to 6 in dishwashing detergents.

## Revendications

1. Mélanges tensioactifs contenant :
(A) 1 à 50 % en poids d'un ou plusieurs tensioactifs anioniques qui contiennent 1 ou 2 radicaux lipophiles de 1 à 22 atomes de C et un radical polaire choisi dans le groupe des radicaux carboxylate, sulfate ou sulfonate et le cas échéant un radical polyoxyalkylène avec un degré moyen d'alcoxylation de 1 à 15.
(B) 0,5 à 15 % en poids d'un ou plusieurs alkylglycosides de formule générale R (G)ₓ où R est un radical alkyle saturé linéaire de 8 à 22 atomes de C et (G)ₓ un radical glycoside ou oligoglycoside avec un degré d'oligomérisation x de 1 à 10,
(C) 0,1 à 7 % en poids d'un copolymère contenant de l'azote avec un poids moléculaire d'environ 15.000 à 1.1500.000 qui comprend,
(i) 99,5 à 45 % molaires d'unités dérivées de vinylpyrrolidone,
(ii) 0,5 à 50 % molaires d'un monomère d'unités dérivées de formule (I)
CH₂=CHR¹-COOR²-NR³R⁴ (I)
où
R¹ est un hydrogène ou un méthyle,
R² est un groupe alkylène de 1 à 20 atomes de C et,
R³ et R⁴ représentent indépendamment l'un de l'autre un groupe alkyle de 1 à 4 atomes de C,
et
(iii) 0 à 50 % molaires d'unités qui sont dérivées d'au moins un monomère éthyléniquement insaturé copolymérisable, différent de (i) et (ii), et
(D) 28 à 98,4 % en poids d'eau, à la condition que,
a) le pH du mélange est 3 à 7, de sorte que le polymère (C) soit temporairement sous la forme cationique,
b) la somme des composants (B) et (C) n'est pas supérieure à la teneur en composant (A) et,
c) le mélange est exempt de polymères et/ou de copolymères contenant de l'azote à caractère cationique permanent.

2. Mélanges tensioactifs selon la revendication 1,
où le copolymère (C) et un copolymère de vinylpyrrolidone et de méthacrylate de diméthylaminoéthyle.

3. Mélanges de tensioactifs selon la revendication 1 ou 2,
où la teneur en tensioactifs anionique (A) est de 4 à 30 % en poids.

4. Mélanges de tensioactifs selon l'une des revendications 1 à 3,
où la teneur en alkylglycoside (B) est 1 à 8 % en poids.

5. Mélanges tensioactifs selon l'une des revendications 1 à 4,
où la teneur en polymère non ionique synthétiques (C) est 0,5 à 5 % en poids.

6. Mélanges tensioactifs selon l'une des revendications 1 à 5,
qui contiennent en plus 0,1 à 10 % en poids d'un sel d'électrolyte (E) minéral.

7. Utilisation des mélanges tensioactifs selon l'une des revendications 1 à 6 dans des formulations cosmétiques rinçages.

8. Utilisation des mélanges tensioactifs selon l'une des revendications 1 à 6 dans des produits cosmétiques de nettoyage tels que les shampooings capillaires, des bains moussants, des produits pour la douche, des savons liquides et analogues.

9. Utilisation des mélanges tensioactifs selon l'une des revendications 1 à 6 dans les produits cosmétiques de soins tels que les rinçages de cheveux, les frictions et analogues.

10. Utilisation de mélanges tensioactifs selon l'une des revendications 1 à 6 dans des produits de rinçage et de nettoyage.
